# EUROPÄISCHE PATENTANMELDUNG

(11) **EP 2 422 792 A1**
(43) Veröffentlichungstag der Anmeldung: **29.02.2012**
(21) Anmeldenummer: 10008875.6
(22) Anmeldetag: 26.08.2010
(51) Int. Cl.: A61K 33/20, A61K 47/02, A61L 2/18, C02F 1/467, C02F 1/461, C02F 103/02

(54) **Elektrochemisch aktivierte Lösung auf Wasserbasis und Verwendung der Lösung**

(71) Anmelder: Caliopa AG, 6314 Unterägeri (CH)
(72) Erfinder: Mathé, Hans-Georg, 6330 Cham (CH)
(74) Vertreter: Tergau & Walkenhorst

(57) **Zusammenfassung**

Eine elektrochemisch aktivierte Lösung auf Wasserbasis, erhältlich durch Elektrolyse von solehaltigem Wasser in einem eine Mehrzahl von Elektrolysezellen (40) umfassenden Elektrolysemodul (2), soll bei besonders hoher Lagerbeständigkeit, also insbesondere auch nach vergleichsweise langer Lagerung von beispielsweise mehr als zwei Jahren, eine besonders hohe bakteriozide oder antibakterielle Wirkung aufweisen. Erfindungsgemäß ist eine derartige Lösung erhältlich durch Elektrolyse von solehaltigem Wasser in einem eine Mehrzahl von Elektrolysezellen (40) umfassenden Elektrolysemodul, bei dem jede Elektrolysezelle (40) jeweils einen einer ersten elektrischen Polarität zugeordneten ersten Elektrodenraum und einen von diesem durch eine Membran (46) getrennten, einer zweiten elektrischen Polarität zugeordneten zweiten Elektrodenraum umfasst, wobei der Elektrolyt den ersten Elektrodenräumen (40) der Elektrolysezellen seriell in der Art einer Reihenschaltung zugeführt wird.

## Beschreibung

### Bezugszeichenliste

- 1: Anlage
- 2: Elektrolysemodul
- 4: Versorgungsleitung
- 6: Wasserenthärterstation
- 8: Venturidüse
- 10: Solebehälter
- 12: Ablaufleitung
- 14: Soleeinspeiseleitung
- 16, 18: Ventil
- 20: Drosselventil
- 22: Ventil
- 24: Abströmleitung
- 26: Vorratsbehälter
- 28: Multiwegeventil
- 30: Abflussleitung
- 40: Elektrolysezelle
- 42: Anodenraum
- 44: Kathodenraum
- 46: Membran
- 48: Verzweigungspunkt
- 50: Zuleitung
- 52: Abströmleitung
- 54: Sammelpunkt
- 56: Überströmleitung
- 58: Überströmleitung
- 60: Abführleitung
- 62: Drosselventil
- 64: Auffangbehälter
- 66: Mengensensor
- 68: Temperatursensor
- 70: pH-Sensor
- 72: Sensor

Die Erfindung betrifft eine elektrochemisch aktivierte Lösung auf Wasserbasis. Sie bezieht sich weiter auf die Verwendung einer derartigen Lösung.

Die Herstellung einer elektrochemisch aktivierten Lösung auf Wasserbasis kann durch Elektrolyse erfolgen. So ist beispielsweise aus der EP 1 728 768 A1 ein System zur Erzeugung einer elektrochemisch aktivierten Salzlösung durch Elektrolyse bekannt, bei dem ein mit einer Kochsalzlösung oder mit Sole beaufschlagter Wasserstrom einer Elektrolysevorrichtung zugeführt wird. Durch die elektrolytische Zersetzung dieses solehaltigen Wasserstroms kann eine elektrochemisch aktivierte wässrige Salzlösung erhalten werden, die einen vergleichsweise hohen Gehalt an Aktivchlor (AC, auch bezeichnet als "freies Chlor", beispielsweise ermittelbar durch amperometrische Messungen) von bis zu 500 mg/L und ein Redoxpotential zwischen +150 mV und +1.350 mV aufweist. Diese elektrochemisch aktivierte Salzlösung ist auf Grund des vergleichsweise hohen Gehalts an Aktivchlor besonders günstig als Desinfektionsmittel, beispielsweise zur Entkeimung von Wasser und/oder wässrigen Lösungen, nutzbar. Darüber hinaus ist die gemäß dem aus der EP 1 728 768 A1 bekannten Konzept erhältliche chemisch aktivierte Salzlösung nach den Angaben in der WO 2009/013019 aber auch besonders günstig als Desinfektionsmittel im allgemeinen Sinne, also beispielsweise für Arbeitsplatten, Tische, Böden, zu Sterilisationszwecken, in Wäschereien oder dergleichen oder auch als Trägermaterial in pharmazeutischen Substanzen nutzbar.

Eine wichtige Kennzahl für die Nutzbarkeit der elektrochemisch aktivierten Salzlösungen in derartigen Anwendungen sind der Gehalt an Aktivchlor (oder "freiem Chlor") sowie der pH-Wert. Für eine besonders hohe bakteriozide oder antibakterielle Wirkung ist hierbei ein möglichst hoher Gehalt an Aktivchlor wünschenswert. Zudem ist dabei gerade im Hinblick auf Verwendungen als Desinfektionsmittel oder für medizinische Zwecke eine besonders gute Lagerbeständigkeit erwünscht, wobei auch nach langer Lagerung der Materialien nur geringe Rückgänge in der antibakteriellen Wirkung auftreten sollten. Bei den bekannten Materialien und Materialzusammensetzungen scheint gerade die Vereinbarkeit dieser Parameter miteinander, also der chemischen Reaktivität, insbesondere ausgedrückt durch den Gehalt an Aktivchlor, und/oder dem Redoxpotential, der Verträglichkeit mit anderen Substanzen oder biologischen Systemen, insbesondere ausgedrückt durch den pH-Wert, und der Lagerbeständigkeit, insbesondere ausgedrückt durch die zeitliche Degradation der Stoffe und den zunehmenden Verlust ihrer bakterioziden oder desinfizierenden Wirkung, problematisch zu sein.

Der Erfindung liegt daher die Aufgabe zu Grunde, eine elektrochemisch aktivierte Lösung auf Wasserbasis, erhältlich durch Elektrolyse, anzugeben, die bei besonders hoher Lagerbeständigkeit, also insbesondere auch nach vergleichsweise langer Lagerung von beispielsweise mehr als zwei Jahren, eine besonders hohe baktiriozide oder antibakterielle Wirkung aufweist.

Diese Aufgabe wird erfindungsgemäß gelöst durch eine elektrochemisch aktivierte Lösung auf Wasserbasis, erhältlich durch Elektrolyse von solehaltigem Wasser in einem eine Mehrzahl von Elektrolysezellen umfassenden Elektrolysemodul, bei dem jede Elektrolysezelle jeweils einen einer ersten elektrischen Polarität zugeordneten ersten Elektrodenraum und einen von diesem durch eine Membran getrennten, einer zweiten elektrischen Polarität zugeordneten zweiten Elektrodenraum umfasst, wobei der Elektrolyt den ersten Elektrodenräumen der Elektrolysezellen seriell in der Art einer Reihenschaltung zugeführt wird.

Wie sich überraschenderweise herausgestellt hat, ist durch eine derartige kaskadenartige Durchströmung einer Mehrzahl von Elektrodenräumen gleicher Polarität in einem Elektrolysemodul mit einer Mehrzahl von Elektrolysezellen die Erzeugung einer hochgradig wirksamen elektrochemisch aktivierten Lösung, charakterisiert durch einen besonders hohen Gehalt an Aktivchlor, möglich. Das zur Erzeugung der elektrochemisch aktivierten Lösung vorgesehene Elektrolysemodul sollte dabei mehrere Elektrolysezellen mit jeweils zumindest zwei Elektrodenräumen umfassen, nämlich einen zum elektrischen Anschluss an den Pluspol einer anzulegenden Elektrolysespannung vorgesehenen Anodenraum und einen zum elektrischen Anschluss an den Minuspol der Elektrolysespannung vorgesehenen Kathodenraum. Die vorgesehene kaskadenartige Durchströmung einer Mehrzahl von der selben Polarität zugeordneten Elektrodenräumen durch den Elektrolyt ist dabei erreichbar, indem die Elektrodenräume der jeweiligen Polarität, also die Anodenräume oder gegebenenfalls auch die Kathodenräume, medienseitig, also bezogen auf die Strömungsführung des Elektrolyten, seriell oder in der Art einer Reihenschaltung hintereinander geschaltet sind.

Eine im Sinne der genannten Parameter chemischer Reaktivität, pH-Neutralität und Lagerbeständigkeit besonders hochwertige elektrochemisch aktivierte Lösung auf Wasserbasis ist dabei erhältlich, indem in besonders vorteilhafter Weiterbildung zusätzlich zur vorgesehenen medienstromseitigen Hintereinander- oder Reihenschaltung der ersten Elektrodenräume des Elektrolysemoduls eine medienstromseitige Parallelschaltung der jeweils zweiten Elektrodenräume der Elektrolysezellen des Elektrolysemoduls vorgesehen ist. Mit anderen Worten: In vorteilhafter Ausgestaltung wird der Elektrolyt einerseits den Elektrodenräumen der ersten Polarität kaskadenartig in Form einer Hintereinanderschaltung und andererseits den Elektrodenräumen der jeweils anderen Polarität in der Art einer Parallelschaltung zugeführt. Durch die sequentielle Durchströmung der Elektrodenräume erfährt der Elektrolyt dabei in der Art einer kaskadierten Behandlung stufenweise in den einzelnen Elektrolysezellen hintereinander mehrfache Behandlungsschritte, so dass eine mehrstufige und damit besonders weit gehende Anreicherung von Ionen im Elektrolyten erreichbar ist.

Zur Herstellung einer hochgradig wirksamen Lösung, charakterisiert durch einen besonders hohen Gehalt an Aktivchlor, ist dabei in besonders vorteilhafter Ausgestaltung die medienseitige Parallelschaltung der Kathodenräume der Elektrolysezellen bei medienseitiger Reihenschaltung der Anodenräume der Elektrolysezellen vorgesehen. Durch eine derartige kaskadierte Behandlung gerade des Anolyten kann eine besonders weit gehende Anreicherung und Aufkonzentration von Chloridionen im Anolyten erreicht werden, so dass gerade im Hinblick auf beispielsweise gewünschte Desinfektionseigenschaften ein besonders hochwertiger Anolyt hergestellt werden kann.

Dabei ist durch die sequentielle Behandlung des Anolyten bei paralleler Medienstromführung des Katholyten gewährleistet, dass bei der Elektrolyse in den jeweiligen Elektrolysezellen das Konzentrationsgefälle zwischen den Ionen im Anolyten einerseits und im Katholyten andererseits in Strömungsrichtung des Anolyten gesehen von Eletrolysezelle zu Elektrolysezelle immer weiter zunimmt. Mit zunehmender Zellenanzahl in der Kaskade ist damit eine zunehmend intensivere Aufkonzentration der Ionen im Anolyten und damit die Bereitstellung eines zunehmend "hochwertigeren" Anolyten möglich.

Eine elektrochemisch aktivierte Lösung mit besonders günstigen Materialeigenschaften hinsichtlich des Gehalts an Aktivchlor und auch hinsichtlich der Lagerbeständigkeit ist erhältlich, indem in besonders vorteilhafter Ausgestaltung die Kathodenräume der Elektrolysezellen medienabströmseitig mit dem Anodenraum der in Strömungsrichtung des Anolyten gesehen ersten Elektrolysezelle verbunden sind. Dabei wird somit der aus den zweiten Elektrodenräumen der Elektrolysezellen abströmende Elektrolyt dem ersten Elektrodenraum der in Strömungsrichtung des Elektrolyten gesehen ersten Elektrolysezelle zugeführt. In dieser besonders bevorzugten medienseitigen Verschaltung führen somit die an die Kathodenräume der Elektrolysezellen angeschlossenen Abströmleitungen vorzugsweise zu einem Sammelpunkt, an dem sie in eine gemeinsame Leitung einmünden. Diese ist ausgangsseitig an den in Strömungsrichtung des Anolyten gesehen ersten Anodenraum der von den Elektrolysezellen anodenseitig gebildeten Kaskade angeschlossen.

In weiterer vorteilhafter Ausgestaltung wird dabei lediglich ein Teilstrom des aus den zweiten Elektrodenräumen der Elektrolysezellen abströmenden Elektrolyts dem ersten Elektrodenraum der in Strömungsrichtung des Elektrolyten gesehen ersten Elektrolysezelle zugeführt. Wie sich überraschenderweise herausgestellt hat, können gerade durch geeignete Einstellung und Veränderung des Verzweigungsverhältnisses in diesem Bereich, also durch gezielte Einstellung des Anteils am Gesamtstrom des aus den Kathodenräumen abströmenden Katholyts, der als Anolyt der weiteren Behandlung zugeführt wird, gezielt wesentliche Parameter des entstehenden Endprodukts, insbesondere der Gehalt an Aktivchlor, der pH-Wert und/oder die Lagerbeständigkeit, beeinflusst werden. Insbesondere kann durch geeignete Einstellung des Verzweigungsverhältnisses die Durchlaufrate und damit die Verweil- und Reaktionszeit des Anolyten in Relation zum Katholyten verändert werden, so dass eine besonders gezielte Aufkonzentration der Ionen erreichbar ist. Des Weiteren ist dadurch bedarfsweise eine Einstellung der Druckverhältnisse und/oder der Strömungsgeschwindigkeiten möglich.

Wie sich überraschenderweise herausgestellt hat, weist die durch das genannte Elektrolyseverfahren erhältliche elektrochemisch aktivierte Lösung auf Wasserbasis besonders wirksame bakteriozide und desinfizierende Eigenschaften auf, wobei dennoch eine außerordentlich geringe Toxizität oder Unverträglichkeit mit menschlichem oder organischen Gewebe gegeben ist. In besonders vorteilhafter Ausgestaltung wird die durch das genannte Elektrolyseverfahren erhältliche elektrochemisch aktivierte Lösung daher in einer pharmazeutischen Zusammensetzung, insbesondere zur Behandlung von Entzündungen, zur Keimreduktion, zur Wundheilung und/oder zur Hygiene, eingesetzt. Dabei kommen insbesondere Verwendungen in Zusammensetzungen für innerliche oder äußerliche Anwendungen an Mensch oder Tier zum Zwecke der Keimreduktion im Blut, in Milchdrüsen und/oder in anderen Körperflüssigkeiten in Galle, Leber, Knochenmark, Drüsen oder Lymphen etc., vorzugsweise bei Entzündungen oder Geschwüren oder auch zum gezielten Töten von Zellen (z. B. Tumorzellen oder virologisch belastete Zellen) in Betracht.

Vorteilhaft ist auch die Verwendung in Zusammensetzungen zur äußerlichen Anwendung an Mensch oder Tier zum Zweck der Keimreduktion, Wundheilung, Prophylaxe und Hygiene, auf Häuten und/oder Schleimhäuten innerlich und äußerlich, z. B. bei Erkältung, Darmkrankheiten wie z. B. Morbus Chron, Magengeschwüre, lokale Antibiotika oder andere geeignete Anwendungen.

Die elektrochemisch aktivierte Lösung kann dabei in der pharmazeutischen Zusammensetzung als aktive Komponente oder Substanz, also als Wirkstoff zur Erzielung eines gewünschten pharmazeutischen Effekts, vorgesehen sein. Hierbei kommt insbesondere eine Nutzung als bakteriozider oder keimtötender Wirkstoff in Betracht. In alternativer vorteilhafter Ausgestaltung kann die elektrochemisch aktivierte Lösung aber auch in der Art einer Trägersubstanz Bestandteil einer pharmazeutischen Zusammensetzung sein, wobei auf Grund der hohen bakterioziden und desinfizierenden Wirksamkeit der als Trägersubstanz vorgesehenen elektrochemisch aktivierten Lösung eine Schutzwirkung für weitere, in der pharmazeutischen Zusammensetzung als Wirkstoff vorgesehene Substanzen oder Komponenten vorgesehen sein kann. Auf Grund der hohen Bakteriozidität bei hoher Lagerbeständigkeit eignet sich die elektrochemisch aktivierte Lösung dabei besonders vorteilhaft als Trägersubstanz für einen Wirkstoff oder eine aktive Substanz, die zu Konservierungs- oder Lagerzwecken besonders wirksam gegen eintretende Verunreinigungen durch Keime oder dergleichen geschützt werden sollte.

Besonders vorteilhaft ist die pharmazeutische Zusammensetzung dabei in der Art eines Mehrkomponentensystems zusammengesetzt, wobei die aktive Substanz oder der Wirkstoff einerseits und die als Trägersubstanz vorgesehene elektrochemisch aktivierte Lösung andererseits in jeweils voneinander separat gehaltenen Phasen vorliegen. Vorteilhafterweise kann dabei die den eigentlichen Wirkstoff oder die aktive Substanz enthaltende Phase in der Art einer Emulsion unter Tröpfchenbildung in die andere, durch die als Trägersubstanz vorgesehene elektrochemisch aktivierte Lösung gebildete Phase eingebettet und von dieser umschlossen sein. Durch diese Phasentrennung ist einerseits sichergestellt, dass die elektrochemisch aktivierte Lösung im Hinblick auf ihre hohe chemische Wirksamkeit die eigentlich als Wirkstoff vorgesehene aktive Substanz nicht zerstört oder beeinträchtigt, wobei andererseits das Vordringen von Keimen oder Verunreinigungen in die von der ersten Phase, also den Wirkstoff, gebildeten Tröpfchen durch das umschließende Trägermaterial wirksam unterbunden wird.

Besonders vorteilhaft eignet sich die elektrochemisch aktivierte Lösung dabei als Trägersubstanz in einer pharmazeutischen Zusammensetzung, in der als aktive Substanz oder Wirkstoff prostaglandine Gewebehormone, Betablocker, eine Substanz zur Verminderung erhöhten Augeninnendrucks und/oder ein ophthalmisches Befeuchtungs- und Trägermittel (insbesondere ein Schmiermittel als Augenbefeuchter, ein Glaucommittel oder dergleichen, vorzugsweise zur Behandlung von Bindehautentzündungen) vorgesehen sind.

Auf Grund ihrer hohen chemischen Wirksamkeit, insbesondere ihrer besonders ausgeprägten Bakteriodizidität, bei hoher Verträglichkeit mit menschlichem Gewebe kann die durch das genannte Elektrolyseverfahren erhältliche elektrochemisch aktivierte Lösung ebenso besonders vorteilhaft als Bestandteil in einem dermatologischen Mittel und/oder als Desinfektionsmittel verwendet werden. Dabei kommen insbesondere Verwendungen in Mitteln zur Wundbehandlung, zur Behandlung von Ulkus (Diabetes), zur Körperpflege, zur Behandlung von Körperschweiss und/oder zur Keimreduktion auf beliebigen Häuten oder Schleimhäuten, in Deodorants, Pickelcremes oder Gleitmitteln, in Mitteln zur Hand-Desinfektion und dergleichen in Betracht.

Weiterhin kann die elektrochemisch aktivierte Lösung vorteilhaft auch in der Art eines allgemeinen Desinfektionsmittels mittelbar, also als Bestandteil, oder unmittelbar verwendet werden, beispielsweise zur Lebensmittelkonservierung oder zu anderen Konservierungszwecken, z. B. von Schnittblumen oder anderen sich durch Keime zersetzenden Stoffe, als äußerliche Anwendung oder als Inhaltsstoff z. B. zur Fruchtwaschung, zur Vernebelung in geschlossenen Räumen wie z. B. Lagerhäusern, zur Keimreduktion in industriellen oder apparativen Prozessen, z. B. in Klimaanlagen, zur Keimreduktion bei der Pflanzenzucht, z. B. in Gewächshäusern, zur Trinkwasseraufbereitung und/oder zur Seuchenbekämpfung.

Die mit der Erfindung erzielten Vorteile bestehen insbesondere darin, dass durch die kaskadenartig oder in der Art einer in mehreren aufeinanderfolgenden Stufen erfolgende elektrochemische Behandlung des Elektrolyten, insbesondere des Anolyten, in einem mehrteiligen Elektrolysemodul der bei der Elektrolyse auftretende Ionenaustausch und damit die lonenbilanz im Elektrolyten derart eingestellt werden kann, dass die solchermaßen elektrochemisch aktivierte Wasserlösung einen besonders hohen Gehalt an Aktivchlor bei besonders guter Lagerbeständigkeit aufweist. Die mit dem genannten Verfahren erhältliche elektrochemisch aktivierte Wasserlösung kann dabei beispielsweise einen Anteil an Aktivchlor von 2000mg/l oder mehr, messbar beispielsweise durch Titrierung mit Iod-Lösungen, und ein Redoxpotential von beispielsweise 900 mV oder gegebenenfalls auch mehr aufweisen. Begünstigt durch den in Kombination mit diesen Parametern zusätzlich erhältlichen pH-Wert von etwa 7 und die dennoch vorhandene Lagerbeständigkeit von zwei Jahren oder mehr ist die solchermaßen erhältliche elektrochemisch aktivierte Lösung auch in Kombination mit anderen Materialien oder Wirkstoffen gut einsetzbar.

Darüber hinaus ist durch geeignete Wahl der Betriebsparameter bei der elektrolytischen Herstellung der elektrochemisch aktivierten Wasserlösung, also insbesondere durch geeignete Wahl der Stromstärke des Elektrolysestroms und der Elektrolysespannung sowie der Durchflussgeschwindigkeit des Medienstroms durch den Reaktor und damit der Verweildauer der Medien im Reaktor der pH-Wert im Endprodukt bei hoher Langzeitstabilität nahezu frei wählbar.

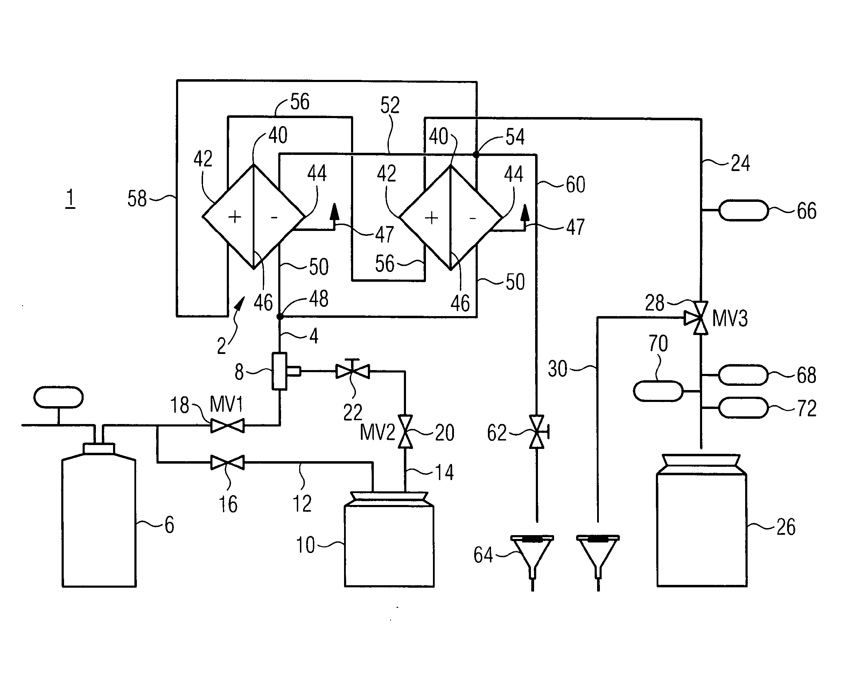

Ein Ausführungsbeispiel der Erfindung wird anhand einer Zeichnung näher erläutert. Darin zeigt die Figur schematisch eine Anlage zur Erzeugung einer elektrochemisch aktivierten Salzlösung durch Elektrolyse.

Die Anlage 1 gemäß der Figur ist zur Erzeugung einer elektrochemisch aktivierten Lösung auf Wasserbasis, insbesondere einer Salzlösung, durch Elektrolyse vorgesehen. Dazu umfasst die Anlage 1 ein Elektrolysemodul 2, dem eingangsseitig über eine Versorgungsleitung 4 ein Elektrolysemedium zuführbar ist. Als Elektrolysemedium ist dabei mit Sole oder einer wässrigen Salzlösung versetztes enthärtetes Wasser vorgesehen. Dazu ist die Zuströmleitung 4 eingangsseitig mit einer Wasserenthärterstation 6 verbunden. Zur Zudosierung der Sole in das enthärtete Wasser ist in die Zuströmleitung 4 eine Venturidüse 8 geschaltet, die ihrerseits eingangsseitig mit einem Solebehälter 10 verbunden ist. Von der Zuführungsleitung 4 zweigt zudem nach der Wasserenthärtestation 6 eine Ablaufleitung 12 ab, über die während einer Inbetriebnahmephase der Anlage 1 der Wasserstrom aus der Wasserenthärterstation 6 unter Umgehung des Elektrolysemoduls 2 der nachfolgenden Komponenten in den Solebehälter 10 abführbar ist. Zur Umschaltung zwischen diesen Betriebszuständen mit dem Dauerbetriebszustand sowie zur dosierten Einspeisung einer vorgebbaren Solemenge in das Elektrolysemedium sind in die Zuführleitung 4, die Ablaufleitung 12 sowie die in die Venturidüse 8 mündende Soleeinspeiseleitung 14 geeignete Ventile 16, 18, 20 und zusätzlich in die Einspeiseleitung 14 ein Drosselventil 20 geschaltet.

Ausgangsseitig, zur Abführung der im Elektrolysemodul 2 hergestellten elektrochemisch aktivierten Salzlösung, ist an das Elektrolysemodul 2 eine Abströmleitung 24 angeschlossen. Diese mündet in einen Vorratsbehälter 26 für die hergestellte Salzlösung oder den Anolyten. Zur vorübergehenden Umgehung des Vorratsbehälters 26 während der Inbetriebnahmephase der Anlage 1 ist in die Ablaufleitung 24 zudem ein Multiwegeventil 28 geschaltet, dessen zweiter Ausgang an eine Abflussleitung 30 angeschlossen ist.

Die Anlage 1 ist zur Herstellung einer elektrochemisch aktivierten Salzlösung mit gerade zur Verwendung als Desinfektionsmittel oder antibakteriellem Wirkstoff in beispielsweise medizinischen oder pharmazeutischen Anwendungen besonders günstigen Eigenschaften, insbesondere mit einem besonders hohen Gehalt an freiem Chlorin von vorzugsweise mehr als 500 mg/L oder ggf. sogar mehr als 2.000 mg/L bei hoher Lagerfähigkeit, ausgelegt. Um dies zu ermöglichen, ist das Elektrolysemodul 2 mehrkomponentig aufgebaut und umfasst eine Mehrzahl von Elektrolysezellen 40, von denen im Ausführungsbeispiel lediglich zwei dargestellt sind; selbstverständlich können analog zu den folgenden Ausführungen aber auch noch weitere Elektrolysezellen 40 vorgesehen sein.

Jede Elektrolysezelle 40 umfasst jeweils einen eine ersten Elektrodenraum bildenden Anodenraum 42 und einen zweiten Elektrodenraum bildenden Kathodenraum 44, die jeweils durch eine Membran 46 voneinander getrennt sind. Das Anlegen einer elektrischen Spannung zwischen einer den jeweiligen Anodenraum 42 begrenzenden Anode einerseits und einen den jeweiligen Kathodenraum 44 begrenzenden Kathode andererseits bewirkt sodann eine Ionenwanderung über die zwischen liegende Membran 46 hinweg, so dass eine zumindest teilweise Dissoziation des im Elektrolysemedium enthaltenen Wassers sowie eine zumindest teilweise Dissoziation der in Form von Sole mitgeführten Salzanteile auftritt. Auf Grund der elektrischen Ladung der Ionen in diesen Materialien führt dieser Ionenwanderungsprozess in Folge der angelegten elektrischen Spannung zu einer Anreicherung von Cl⁻ und OH⁻ im jeweiligen Anodenraum 42 und zu einer Anreicherung von H⁺ und Na⁺ im jeweiligen Kathodenraum 44. Zur bedarfsweisen Abführung von demzufolge im jeweiligen Kathodenraum 44 oder zweiten Elektrodenraum angereichertem Wasserstoffgas ist der Kathodenraum 44 jeweils mit einer Entgasungsleitung 47 verbunden. Diese bildet, ggf. in Kombination mit im Kathodenraum 44 angeordneten Mitteln zur Bläschenerzeugung oder Gasabscheidung wie beispielsweise Verwirbler oder dergleichen, ein Entgasungsmodul für den jeweiligen Kathodenraum 44.

Die gewünschten, qualitativ hochwertigen Eigenschaften der elektrochemisch aktivierten Salzlösung werden in der Anlage 1 insbesondere durch eine spezifische Führung der Medienströme im Elektrolysemodul 2 erreicht. Dabei ist in der Anlage 1 gemäß dem Ausführungsbeispiel, die gezielt auf die Bereitstellung von elektrochemisch hochgradig aktiviertem Anolyt ausgerichtet ist, eine kathodenseitig im Wesentlichen parallele Medienstromführung mit einer anodenseitig im Wesentlichen seriellen Medienstromführung kombiniert. Alternativ oder bei abweichendem Auslegungsziel könnte aber auch eine anodenseitig im Wesentlichen parallele mit einer kathodenseitig im Wesentlichen seriellen Medienstromführung kombiniert sein. Darüberhinaus können auch noch weiterführende Kombinationen dieser medienseitigen Verschaltungen vorgesehen sein.

Im Einzelnen ist dabei in der Anlage 1 im Ausführungsbeispiel in der Zuströmleitung 4 ein Verzweigungspunkt 48 vorgesehen, von dem aus in der Art einer parallelen Medienzuführung in die Kathodenräume 44 der Elektrolysezellen 40 mündende Zuleitungen 50 abgehen. Ausgangsseitig der Kathodenräume 44 sind Abströmleitungen 52 vorgesehen, die in einem Sammelpunkt 54 zusammengeführt sind, so dass sich insgesamt eine medienseitig parallele Verschaltung der Kathodenräume 44 ergibt.

Anodenseitig ist hingegen eine Reihen- oder Hintereinanderschaltung der Anodenräume 42 für den Anolyten vorgesehen. Dazu ist der in Strömungsrichtung des Anolyten gesehen dem ersten Elektrolysemodul 40 zugeordnete Anodenraum 42 ausgangsseitig über eine Überströmleitung 56 mit der Eingangsseite des nachfolgenden Anodenraums 42 verbunden. Dieser ist seinerseits ausgangsseitig an die Abströmleitung 24 angeschlossen, so dass sich in der Art einer mehrstufigen oder kaskadenartigen Ausführung eine Hintereinanderschaltung der Anodenräume 42 bzgl. des Anolyten ergibt.

Zudem ist in der Anlage 1 vorgesehen, den aus den Kathodenräumen 44 abströmenden Katholyten als Anolyt in die hintereinander geschalteten Anodenräume 42 einzuspeisen. Dazu ist der Sammelpunkt 54 für den Katholyten über eine Überströmleitung 58 mit dem Anodenraum 42 der in Strömungsrichtung des Anolyten gesehen ersten Elektrolysezelle 40 verbunden.

Von der Überströmleitung 58 zweigt im Sammelpunkt 54 zudem eine Abführleitung 60 ab, über die ein in seiner Menge über ein Drosselventil 62 einstellbarer Teilstrom des Katholyten einem Abwassersystem oder einem Auffangbehälter 64 zugeführt werden kann. Mit dieser Anordnung ist es möglich, eine einstellbare Teilmenge des aus den Kathodenräumen 44 abströmenden Katholyten als Anolyt der Kaskade aus Anodenräumen 42 zuzuführen. Damit können unter anderem die individuellen Reaktionsparameter wie beispielsweise Druck und Fließgeschwindigkeit in den Anodenräumen 42 geeignet beeinflusst werden, und zudem kann die Menge des in diesem Anwendungsfall als "Abfallprodukt" anfallenden Katholyts besonders gering gehalten werden.

Zur Überprüfung der Materialeigenschaften des hergestellten Anolyten sowie zur Mengenmessung sind im Übrigen in die Abströmleitung 24 eine Anzahl von Sensoren, insbesondere ein Mengensensor 66, ein Temperatursensor 68, ein pH-Sensor 70 sowie ein Sensor 72 zur Messung des Redoxpotentials, geschaltet.

Wie bereits erwähnt ist die Anlage 1 gemäß der Figur gezielt für die Bereitstellung von besonders hochwertigem Anolyt durch eine kaskadenartige medienstromseitige Hintereinanderschaltung der Anodenräume 42 bei medienstromseitiger Parallelschaltung der Kathodenräume 44 ausgelegt. Eine im Hinblick auf ihre chemischen Eigenschaften, insbesondere den Anteil an Aktivchlor und das Redoxpotential, sowie auf die Lagerbeständigkeit besonders hochwertige elektrochemisch aktivierte Lösung ist durch den Betrieb der Anlage 1 beispielsweise erhältlich, indem über die Zuströmleitung 4 solehaltiges Wasser über den Verzweigungspunkt 58 parallel in die Kathodenräume 44 eingespeist wird. Die Konzentration der Sole im enthärteten Wasser wird dabei über das Ventil 22 und/oder die Venturidüse 8 geeignet und abhängig vom gewünschten Salzgehalt bzw. der Ionenkonzentration im Endprodukt eingestellt, und im Elektrolysemodul wird der Anolyt mit einer geeignet gewählten Durchflussrate geführt. Nach einer Verweildauer wird der Katholyt dann in den Kathodenräumen 44 über die jeweils benachbarten Membranen 46 mit Ionen beaufschlagt, wobei zwischen der jeweiligen Anode und der jeweiligen Kathode die Elektrolysespannung angelegt wird.

Anschließend wird der Katholyt aus den Kathodenräumen 44 wieder abgeführt und dem Sammelpunkt 54 zugeführt. Nach dem Sammelpunkt 54 wird der Katholyt in zwei Teilströme aufgeteilt, wobei ein erster Teilstrom des Katholyten von etwa 50 bis 95% des Gesamtstroms, vorteilhafterweise von etwa 90% des Gesamtstroms, über die Überströmleitung 58 dem Anodenraum 42 der in Strömungsrichtung des Anolyten gesehen ersten Elektrolysezelle 40 zugeführt wird. Der zweite Teilstrom des Katholyten, entsprechend etwa 5 bis 50% des Gesamtstroms, vorzugsweise etwa 10% des Gesamtstroms, wird hingegen über die Abführleitung 60 dem Auffangbehälter 64 zugeführt.

Der erste Teilstrom wird anschließend, ausgehend von dem Anodenraum 42 der in Strömungsrichtung des Anolyten gesehen ersten Elektrolysezelle 40, kaskadenartig als Anolyt durch die medienstromseitig in Reihe geschalteten Anodenräume 42 der Elektrolysezellen 40 geführt und dabei in der Art einer mehrstufigen Behandlung mehreren aufeinanderfolgenden elektrochemischen Aktivierungsschritten unterzogen.

Die auf diese Weise erhältliche elektrochemisch aktivierte Lösung zeichnet sich insbesondere durch einen vergleichsweise hohen Gehalt an Aktivchlor bei hoher Lagerbeständigkeit aus. Der Salzgehalt in den Endprodukten, also im hergestellten Anolyt ebenso wie im hergestellten Katholyt, kann dabei durch entsprechend eingestellte Zudosierung bei der Einspeisung der Sole aus dem Solebehälter 10 in den Strom enthärteten Wassers bestimmungsgemäß (beispielsweise wahlweise als "niedrige Konzentration" oder auch als "hohe Konzentration") eingestellt werden. Insbesondere kann durch geeignete Zumischung der Sole in den Wasserstrom die Leitfähigkeit des Endprodukts geeignet vorgegeben werden; die Leitfähigkeit kann dabei für einen Anolyten vergleichsweise niedriger Konzentration an Aktivchlor auf etwa 10 mS/cm und für einen Anolyten vergleichsweise hoher Konzentration an Aktivchlor auf etwa 18 mS/cm eingestellt werden.

Anschließend kann über eine geeignete Einstellung der Fließgeschwindigkeit der Medien im Reaktor, also im Elektrolysemodul 2, die "Behandlungsdauer" von Anolyt einerseits und Katholyt andererseits geeignet eingestellt werden, wodurch insbesondere die gezielte Einstellung des pH-Werts der Endprodukte möglich ist. Dabei sollte die Betriebstemperatur in den Medienströmen, die sich abhängig von der Stromstärke des Elektrolysestroms einstellt, nicht zu hoch gewählt werden, um eine hohe Produktqualität sicherzustellen. Sowohl für Anolyten niedriger Konzentration als auch für Anolyten hoher Konzentration lassen sich dabei pH-Werte vorzugsweise im Bereich von 7,0 bis 7,6 einstellen, wobei der entstehende Anolyt eine klare, farblose Flüssigkeit mit chlorbasiertem Geruch und einem Redoxpotential von beispielsweise 850 bis 950 mV (potentiometrisch gemessen) sein kann. Vorzugsweise werden aber auch Redoxpotentiale von mehr als 1500 mV, insbesondere auch von mehr als 2000 mV, eingestellt.

Der durch jodometrische Titration ermittelbare Wirkstoffgehalt (oder Anteil an Aktivchlor) beträgt dabei bei nach dem Ausführungsbeispiel hergestelltem Anolyt für Anolyt niedriger Konzentration (vorzugsweise verwendbar als Desinfektionsmittel und charakterisiert durch eine Leitfähigkeit von etwa 10 mS/cm) etwa 600 bis 800 ppm und für Anolyt hoher Konzentration (vorzugsweise verwendbar als medizinischer oder pharmazeutischer Wirkstoff und charakterisiert durch eine Leitfähigkeit von etwa 16 bis 20 mS/cm) etwa 800 bis 950 ppm.

Über diese Ausführungsbeispiele hinaus kann der Anolyt beispielhaft auch mit folgenden Kriterien und/oder charakteristischen Parametern hergestellt werden:
- Der Elektrolyt charakterisiert sich durch ein in Wasser gelöstes Meta-Gleichgewicht aus Ionen aus Salz (Na, Cl), Wasser (H2O, OH, H30), ClOx und ggf. Cl2Oy, deren Säuren und Basen, deren Anionen und Kationen und deren sich gegenseitig bedingende Lösungsspezies.
- Je nach Einstellung der Betriebsparameter im Herstellungsprozess, insbesondere Durchflussraten der Medien, Betriebsspannungen und -ströme, Konzentrationsvorgaben, können für den Gehalt an Aktivchlor Werte zwischen beispielsweise 400 und 2000 mgAC/l, ggf. auch mehr als 2000 mgAC/l, eingestellt werden.
- Der pH-Wert ist einstellbar zwischen 2 und 14, vorzugsweise wird ein Wert zwischen 6 und 8 eingestellt.
- Die Leitfähigkeit kann im Bereich zwischen 14 mS (untere Grenze 4 mS) und 25 mS liegen und wird vorzugsweise auf einen Wert im Bereich von 10 mS (Oberfläche) und 18 mS (isotonisch) und 22 mS (Pharmaanwendungen) eingestellt.
- Der Salzgehalt kann auf einen Wert im Bereich von 0,1 g/l bis 15 g/l eingestellt werden; vorzugsweise wird ein Wert im (isotonischen) Bereich von 5g/l bis etwa 9 g/l gewählt.

## Patentansprüche

1. Elektrochemisch aktivierte Lösung auf Wasserbasis, erhältlich durch Elektrolyse von solehaltigem Wasser in einem eine Mehrzahl von Elektrolysezellen (40) umfassenden Elektrolysemodul (2), bei dem jede Elektrolysezelle (40) jeweils einen einer ersten elektrischen Polarität zugeordneten ersten Elektrodenraum und einen von diesem durch eine Membran (46) getrennten, einer zweiten elektrischen Polarität zugeordneten zweiten Elektrodenraum umfasst, wobei der Elektrolyt den ersten Elektrodenräumen der Elektrolysezellen (40) seriell in der Art einer Reihenschaltung zugeführt wird.

2. Elektrochemisch aktivierte Lösung nach Anspruch 1, wobei der Elektrolyt den zweiten Elektrodenräumen der Elektrolysezellen (40) parallel zugeführt wird.

3. Elektrochemisch aktivierte Lösung nach Anspruch 1 oder 2, wobei die ersten Elektrodenräume der Elektrolysezellen (40) jeweils als Anodenräume (42) und die zweiten Elektrodenräume der Elektrolysezellen (40) jeweils als Kathodenräume (44) ausgestaltet sind.

4. Elektrochemisch aktivierte Lösung nach einem der Ansprüche 1 bis 3, wobei aus den zweiten Elektrodenräumen der Elektrolysezellen (40) abströmender Elektrolyt dem ersten Elektrodenraum der in Strömungsrichtung des Elektrolyten gesehen ersten Elektrolysezelle (40) zugeführt wird.

5. Elektrochemisch aktivierte Lösung nach Anspruch 4, wobei lediglich ein Teilstrom des aus den zweiten Elektrodenräumen der Elektrolysezellen (40) abströmenden Elektrolyts dem ersten Elektrodenraum der in Strömungsrichtung des Elektrolyten gesehen ersten Elektrolysezelle (40) zugeführt wird.

6. Pharmazeutische Zusammensetzung, insbesondere zur innerlichen oder äusserlichen Behandlung von Entzündungen, zur Keimreduktion, zur Wundheilung und/oder zur Hygiene, umfassend eine elektrochemisch aktivierte Lösung nach einem der Ansprüche 1 bis 5.

7. Pharmazeutische Zusammensetzung, einerseits umfassend eine aktive Substanz, beispielsweise prostaglandine Gewebehormone, Betablocker, eine Substanz zur Verminderung erhöhten Augeninnendrucks, und/oder ein ophthalmisches Befeuchtungsmittel, und andererseits umfassend eine Trägersubstanz basierend auf einer elektrochemisch aktivierten Lösung nach einem der Ansprüche 1 bis 5, wobei die aktive Substanz in derselben oder in einer von der elektrochemisch aktivierten Lösung separaten Phase vorliegt.

8. Dermatologisches Mittel, insbesondere zur Behandlung von Wunden, Ulkus (Diabetes) und/oder anderen Hauterkrankungen, umfassend eine elektrochemisch aktivierte Lösung nach einem der Ansprüche 1 bis 5.

9. Desinfektionsmittel, umfassend eine elektrochemisch aktivierte Lösung nach einem der Ansprüche 1 bis 5.

10. Verwendung einer elektrochemisch aktivierten Lösung nach einem der Ansprüche 1 bis 5 in einer pharmazeutischen Zusammensetzung.

11. Verwendung einer elektrochemisch aktivierten Lösung nach einem der Ansprüche 1 bis 5 in einem Desinfektionsmittel.
